# EUROPEAN PATENT SPECIFICATION

(11) **EP 4 304 578 B1**
(45) Date of publication and mention of the grant of the patent: **06.05.2026**
(21) Application number: 22713921.9
(22) Date of filing: 10.03.2022
(51) Int. Cl.: A61K 31/20, A61P 31/12, A61P 31/16

(54) **PELARGONIC ACID FOR USE AGAINST AFRICAN SWINE FEVER VIRAL INFECTIONS**
PELARGONSÄURE ZUR VERWENDUNG GEGEN VIRALE INFEKTIONEN DER AFRIKANISCHEN SCHWEINEPEST
ACIDE PÉLARGONIQUE DESTINÉ À ÊTRE UTILISÉ CONTRE LES INFECTIONS VIRALES DE LA PESTE PORCINE AFRICAINE

(30) Priority: 10.03.2021 BE 202105180
(43) Date of publication of application: 17.01.2024
(73) Proprietor: Nutrition Sciences N.V., 9031 Drongen (BE)
(72) Inventor: LANNOO, Kobe, Drongen DRONGEN (BE); BRUGGEMAN, Geert, 9031 DRONGEN (BE); BRUGGER, Roland, 9031 DRONGEN (BE)
(74) Representative: Brantsandpatents bv
(86) International application number: PCT/EP2022/056248
(87) International publication number: WO 2022/189588

(56) References cited:
- EP-A1- 1 123 701
- WO-A1-03/043441
- WO-A1-2013/059012
- CN-A- 106 234 770
- BORCA MANUEL V. ET AL: "Development of a Highly Effective African Swine Fever Virus Vaccine by Deletion of the I177L Gene Results in Sterile Immunity against the Current Epidemic Eurasia Strain", JOURNAL OF VIROLOGY, vol. 94, no. 7, 17 March 2020 (2020-03-17), US, XP093309205, ISSN: 0022-538X, Retrieved from the Internet <URL:https://journals.asm.org/doi/pdf/10.1128/JVI.02017-19> DOI: 10.1128/JVI.02017-19
- VIETNAM+ (VIETNAMPLUS): "Vietnam successfully produces vaccine against African swine fever | Vietnam+ (VietnamPlus)", 1 June 2022 (2022-06-01), pages 1 - 5, XP093309207, Retrieved from the Internet <URL:https://en.vietnamplus.vn/vietnam-successfully-produces-vaccine-against-african-swine-fever-post229505.vnp>

## Description

### FI ELD OF THE I NVENTI ON

The present invention relates to mitigants and treatments for viral infections in animals such as pigs.

### BACKGROUND

In the recent years, outbreaks of several viruses have challenged the agricultural industry such as the swine industry worldwide. In 2015 the spread of Porcine Epidemic Diarrhea (PED) shocked the industry, now African Swine Fever (ASF) is changing the global pig production landscape. African Swine Fever Virus (ASFV) is a contagious, rapidly spreading, transboundary animal disease and a major threat to pork production globally. Handling these kind of threats needs a very broad approach including biosecurity measurements, veterinary precautions, nutritional adaptations and feed safety measurements to stop the disease from spreading.

Learnings from previous foreign animal disease outbreaks have shifted the focus from diagnosis and treatment to prevention and control. Keeping pathogens at bay is the first step to an effective on-farm biosecurity program. An often-overlooked aspect in biosecurity planning, however, is the role of feed and feed ingredients. In particular, it is known that feed may carry pathogens that are detrimental to animal health and welfare. As such, spreading of viral particles via the feed is an example of growing concern. To this purpose antiseptic products such as formaldehyde are commonly used to disinfect the feed.

WO 2016 081 716 discloses the use of chemical mitigants to prevent or decrease Porcine Epidemic Diarrhea virus (PEDv) and/or Salmonella bacteria in animal feed. WO 2019 169 256 describes chemical mitigants to combat African swine fever virus or classical swine fever virus. WO 2009 150 281 and CN 106 234 770 discuss the use of conjugated fatty acids for combatting viral infections in animals. EP 1 123 701 discusses the use of fatty acids for treating fish parasitic diseases and WO 2003 043 441 and WO 2013 059 012 describe the use of medium-chain fatty acids (MCFAs) as antimicrobial agents.

Despite these proposed solutions, the industry to date lacks adequate methods to prevent the spreading of viral particles and consequently to prevent viral infections in animals, especially pigs. More specifically, there is a need for treatments that are also effective at mitigating viral presence in animal feed, feed ingredients and drinking water, while also being safe for oral administration for animals, especially pigs.

### SUMMARY OF THE INVENTION

The present invention is defined in the appended claims. Embodiments not falling within the scope of the appended claims do not form part of the invention. Any references in the description to methods of treatment refer to the compounds, pharmaceutical compositions and medicaments of the present invention for use in a method for treatment of the human (or animal) body by therapy. The invention thereto aims to provide pelargonic acid (C9) for use in the treatment, suppression and/or prevention of viral infections in animals according to claim 1.

In a second aspect, the present invention relates to a method of inhibiting or mitigating viral infections in animal feed, feed ingredients or drinking water according to claim 10. The method comprises dosing pelargonic acid (C9) to said animal feed, feed ingredient or drinking water.

Another aspect of the invention relates to a method of lowering the viral titer in an animal feed, feed ingredient or drinking water according to claim 14, wherein the method comprises adding an effective dose of pelargonic acid to said animal feed, feed ingredient or drinking water.

### DESCRI PTI ON OF FIGURES

The following description of the figures of specific embodiments of the invention is merely exemplary in nature.
Fig. 1 shows a graph relating to data in support of the susceptibility of African Swine Fever Virus (ASFV) to pelargonic acid in animal feed.
Fig. 2 shows a graph relating to data in support of the susceptibility of African Swine Fever Virus (ASFV) to pelargonic acid in animal drinking water.

### DETAI LED DESCRI PTI ON OF THE INVENTION

The present invention concerns the use of pelargonic acid for treating viral infections in animals, preferably pigs as well as methods for inhibiting or lowering the viral titer in feed, feed ingredients or drinking waters of animals. Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
The term "medium-chain fatty acid" or "MCFA" as used herein, refers to fatty acids with a medium-chain length, wherein the fatty acids may be saturated or unsaturated. In the present invention, the longest continuous chain of an MCFA can consist of 5 to 12 carbon atoms, for example, valeric acid (C5), caproic acid (C6), caprylic acid (C8), pelargonic acid (9), capric acid (C10) or lauric acid (C12). The term MCFA also refers to medium-chain fatty acids that are chemically modified, and to medium-chain fatty acids that are provided with side-chains, such as, without limitation, one or more alkyl groups, preferably C1-C10 alkyl groups.

As described herein, the term "derivative of a an MCFA or pelargonic acid" refers to a fatty acid chain of which the carboxyl group is reversibly converted to a different group, preferably, but without limitation, to an amide, salt, ester or glyceride. As described herein, the term "free fatty acids" refers to fatty acids that are not converted into a salt or a derivative (such as an amide, ester or glyceride). The use of esters and salts, for example, prevents the diffusion of bad odors, which may occur when the free fatty acids are used.

Derivatives needs to be interpreted as being the various forms of the free MCFA or pelargonic acid:
- The term "medium-chain fatty acid (MCFA) derivative" refers to a fatty acid chain of which the carboxyl group is reversibly converted to a different group, preferably, but without limitation, to an amide, salt, ester or glyceride such as, for example, a mono-, di- or tri-glyceride. According the current invention the primary MCFA is pelargonic acid (C9).
- When circulating or unbound, fatty acids are known as non-esterified fatty acids (NEFAs) or free fatty acids (FFAs). Pelargonic acid can occur in the FFA form.
- MCFAs in general and pelargonic acid specifically may exist as an ester, wherein the ester can be classified as triglyceride, phospholipid, or cholesteryl ester.
- MCFAs in general and pelargonic acid specifically can occur as a metal salt conjugate, such as a sodium, potassium or calcium salt of pelargonic acid, wherein a sodium salt is the most prevalent. The sodium salt of pelargonic acid is called sodium nonanoate.

The term "antiviral in vitro assay" as used herein refers to any method suitable to determine potential efficacy of an antiviral substance, compound or composition towards a chosen virus or viral family. Known methods comprise TCID50 assays, EC50/CC50 assays, plaque assays, HAI (hemagglutination inhibition) assays and ELISA/Luminex.

Cytopathic effect or cytopathogenic effect (abbreviated CPE) is to be understood as structural changes in host cells that are caused by viral invasion. The infecting virus causes lysis of the host cell or when the cell dies without lysis due to an inability to reproduce. Both of these effects occur due to CPEs. If a virus causes these morphological changes in the host cell, it is said to be cytopathogenic. Common examples of CPE include rounding of the infected cell, fusion with adjacent cells to form syncytia, and the appearance of nuclear or cytoplasmic inclusion bodies. CPEs and other changes in cell morphology are only a few of the many effects by cytocidal viruses. When a cytocidal virus infects a permissive cell, the viruses kill the host cell through changes in cell morphology, in cell physiology, and the biosynthetic events that follow. These changes are necessary for efficient virus replication but at the expense of the host cell.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the current inventions disclose Pelargonic acid (C9) for use in the treatment or prevention of viral infections in animals, from the Family of Suidae, including pigs, hogs or boars, as claimed.

The consequences of viral infections in animals, especially livestock and farm animals, have a vast economic effect. Although variable from continent to continent and from country to country, the need for new compounds for use in the treatment or prevention of viral infections in animals remains imminent. For most viral infections, treatments can only help with symptoms while waiting for the immune system to fight off the virus. There are antiviral medicines to treat some viral infections and the development of vaccines can help prevent from getting many viral diseases, however against many viruses there is currently no effective vaccine. The current invention discloses pelargonic acid (C9) as novel therapeutic agent in the treatment or prevention of viral infections in animals, as claimed.

Preferably the viral infections that are treated or prevented cause problems of the gastrointestinal tract, such as diarrhea, or of the respiratory tract such as pneumonia or pneumonia-like symptoms. Gastrointestinal and respiratory tract infections account for the bulk of all infection-related mortality in animals. Pelargonic acid represent a natural alternative that poses essentially no risk to the animals or the environment, while still effectively treating or preventing gastrointestinal and respiratory tract infections.

Common viral infections are Avian Influenza A virus, African Swine Fever virus (ASFV), Porcine Epidemic Diarrhoea Virus (PEDV), Porcine Reproductive and Respiratory Syndrome Virus (PRRS) or Classical Swine Fever Virus.

Avian influenza A, known informally as avian flu or bird flu, is a variety of influenza caused by viruses adapted to birds. The type with the greatest risk is highly pathogenic avian influenza (HPAI). Bird flu is similar to swine flu, dog flu, horse flu and human flu as an illness caused by strains of influenza viruses that have adapted to a specific host. Out of the three types of influenza viruses (A, B, and C), influenza A virus is a zoonotic infection with a natural reservoir almost entirely in birds. Avian influenza, for most purposes, refers to the influenza A virus. Avian influenza strains are divided into two types based on their pathogenicity: high pathogenicity (HP) or low pathogenicity (LP). The most well-known HPAI strain, H5N1, was first isolated from a farmed goose in Guangdong Province, China in 1996, and also has low pathogenic strains found in North America.

African Swine Fever Virus (ASFV) is a DNA virus belonging to the Asfarviridae family.

At least 22 types exist which enables outbreaks to be traced to source. It is extremely resistant to putrefaction and sunlight, and can persist in refrigerated meat and carcasses for up to 6 months and for much longer when frozen. Infection is spread from pig to pig usually by aerosol from infected discharges and feces, by the bites of soft ticks, the bites of lice and flies and by direct inoculation from contaminated syringes. Infection can also be spread on contaminated implements and during transport. The virus can survive in carrier pigs, in infected buildings and for up to 4 years in infected Ornithodorus ticks. African Swine Fever is highly contagious and infection spreads rapidly through a unit, with clinical signs of fever beginning 4-5 days after infection and causing fever followed by dullness, breathing difficulty, vomiting, coughing, nasal and ocular discharge, abortion in pregnant sows, cyanosis of the extremities and death within 7 days.

Porcine Epidemic Diarrhea virus (PEDv) is a coronavirus that is responsible for the death of over 25 million pigs worldwide. The virus affects pigs of all stages, but is particularly catastrophic to suckling pigs because of their less developed digestive tracts, which cannot absorb nutrients in the small intestine. The virus causes severe diarrhea and vomiting. The virus is not transmissible to other species, and the pork is safe to consume. However, poor animal health and performance has led to a low pork supply, which is the source of record pork prices for consumers. The virus is transmitted by fecal-oral contamination, but epidemiological and controlled research evidence has confirmed swine feed or ingredients are another vector of transmission.

Betaarterivirus suid 1, formerly Porcine reproductive and respiratory syndrome virus (PRRSV), is a virus that causes a disease of pigs, called porcine reproductive and respiratory syndrome (PRRS), also known as blue-ear pig disease. This economically important, panzootic disease causes reproductive failure in breeding stock and respiratory tract illness in young pigs. Initially referred to as "mystery swine disease" and "mystery reproductive syndrome", it was first reported in 1987 in North America and Central Europe. The disease costs the United States swine industry around $644 million annually, and recent estimates in Europe found that it costs almost 1.5b€ every year. PRRSV is a small, enveloped RNA virus. It contains a single-stranded, positive-sense, RNA genome with a size of approximately 15 kilobases. PRRSV is a member of the genus Arterivirus, family Arteriviridae, order Nidovirales.

Classical swine fever virus (CSFV) is a single-stranded RNA virus in the Flaviviridae family. CSFV is closely related to the ruminant pestiviruses that cause bovine viral diarrhoea and border disease. The effect of different CSFV strains varies widely, leading to a wide range of clinical signs. Highly virulent strains correlate with acute, obvious disease and high mortality, including neurological signs and hemorrhages within the skin. Less virulent strains can give rise to subacute or chronic infections that may escape detection, while still causing abortions and stillbirths. In these cases, herds in high-risk areas are usually serologically tested on a thorough statistical basis. Infected piglets born to infected but subclinical sows help maintain the disease within a population. Other signs can include lethargy, fever, immunosuppression, chronic diarrhoea, and secondary respiratory infections. The incubation period of CSF ranges from 2 to 14 days, but clinical signs may not be apparent until after 2 to 3 weeks. Preventive state regulations usually assume 21 days as the outside limit of the incubation period. Animals with an acute infection can survive 2 to 3 months before their eventual death. Eradicating CSF is problematic. Current programs revolve around rapid detection, diagnosis, and slaughter. Vaccination is only used where the virus is widespread in the domestic pig population and/or in wild or feral pigs. In the latter case, a slaughter policy alone is usually impracticable. Instead, countries within the EU have implemented hunting restrictions designed to limit the movement of infected boars, as well as using marker and emergency vaccines to inhibit the spread of infection. Possible sources for maintaining and introducing infection include the wide transport of pigs and pork products, as well as endemic CSF within wild boar and feral pig populations.

The inventors have found that supplying pelargonic acid (C9) to feed, feed ingredients or the drinking water of animals may prevent and treat diseases caused by viral infections. While the mode of action is unclear, it is believed that the viral particles present therein are eradicated by the pelargonic acid.

Mixtures of MCFA have in the past been described as a potential treatment against bacterial and viral infections. However, the inventors found that pelargonic acid was especially effective in treating viral infections in animals and provided better results when being compared to other MCFAs or their mixtures.

Pelargonic acid can be conveniently used in the treatment, suppression and/or prevention of viral infections as claimed, as it can be administered orally to the animals or mixed with their feed, feed ingredients or drinking water. Oral administration is attained by feeding pelargonic acid to the animals or by allowing the animals to drink the drinking water. This method of administration provides the advantage that it is easy to perform and it does not impose the burden of large investments for novel equipment on the farmer.

The feed can comprise other well-known ingredients so as to provide a nutritionally balanced complete food, including, but not limited to, plant matter, e.g., flour, meal, starch or cracked or processed grain produced from a crop plant such as wheat or other cereals, alfalfa, corn, oats, potato, rice, soybeans or other legumes; cellulose in a form that may be obtained from wood pulp, grasses, plant leaves, and waste plant matter such as rice or soy bean hulls, or corn cobs; animal matter, e.g., fish or crustacean meal, oil, protein or solubles and extracts, krill, meat meal, bone meal, feather meal, blood meal, or cracklings; algal matter; yeast; bacteria; vitamins, minerals, and amino acids; organic binders or adhesives; and chelating agents and preservatives.

Oral administration can also be performed by admixing the animal drinking water with pelargonic acid. Accordingly, the invention provides a drinking water comprising pelargonic acid for use as described above.

In one embodiment, pelargonic acid (or derivatives thereof) is added at a concentration of between 1 and 10.000 ppm to said feed, feed ingredients or drinking water, preferably between 50 ppm and 10000 ppm by weight, preferably between 100 ppm and 10000 ppm by weight, preferably between 200 ppm and 5000 ppm by weight, more preferably between 250 and 5000 ppm by weight, even more preferably between 250 and 3000 ppm or between 1000 and 5000 ppm by weight, such as 1250 ppm, 1500 ppm, 2000 ppm or 3750 ppm based on the total weight of said feed, feed ingredients or drinking water.

The inventors have found that pelargonic acid as described above shows optimal preventive and curative properties towards viral infections responsible for causing viral disease.

In one embodiment, pelargonic acid is combined with one or more other organic acids. An organic acid is an organic compound with acidic properties. The most common organic acids are the carboxylic acids, whose acidity is associated with their carboxyl group -COOH. Sulfonic acids, containing the group -SO2OH, are relatively stronger acids. Alcohols, with -OH, can act as acids but they are usually very weak. The relative stability of the conjugate base of the acid determines its acidity. Other groups can confer acidity, usually weakly: the thiol group -SH, the enol group, and the phenol group. In biological systems, organic compounds containing these groups are generally referred to as organic acids.

According to a further or another embodiment, pelargonic acid is combined with one or more organic acids chosen from the group of short chain fatty acids and their derivatives, medium chain fatty acids and their derivatives, propionic acid, acetic acid, lactic acid, formic acid, citric acid, oxalic acid, malic acid, or combinations thereof. Short-chain fatty acids (SCFA) are fatty acids with aliphatic tails of five or fewer carbons (e.g. butyric acid). Medium-chain fatty acids (MCFA) are fatty acids with aliphatic tails of 6 to 12 carbons, which can form medium-chain triglycerides. The organic acids comprised herein result in a composition which is soluble in water and therefore is ideally suited for oral administration by admixing the composition with animal drinking water. By preference, said organic acids are present in the composition in a concentration of between 60% and 80% on the total weight of the composition.

In one embodiment, pelargonic acid is combined with additional medium chain fatty acids (MCFAs) or their derivatives, wherein said MCFAs are chosen from the group of valeric acid (C5), caproic acid (C6), caprylic acid (C8), capric acid (C10), lauric acid (C12), derivatives thereof or combinations thereof.

In one embodiment, the ratio between C9 and the sum of the other MCFAs is between 50:1 to 1:1, preferably 45:1 to 1:1, preferably 40:1 to 1:1, preferably 35:1 to 1:1, preferably 30:1 to 1:1, preferably 25:1 to 1:1, preferably 20:1 to 1:1, preferably 15:1 to 1:1, more preferably 10:1 to 1:1.

Due to this outstanding effectiveness on prevention, suppression and/or treatment of viral infections, the current invention leads to higher revenues for the farmers while reducing the costs of disease prevention, treatment and/or suppression.

In a further or other embodiment, said pelargonic acid is further mixed with vitamins, minerals, trace elements or a combination thereof. Vitamins include but are not limited to Vitamin A, Vitamin D3, Vitamin E, Vitamin K3, Thiamin, Riboflavin, Panthothenic acid, Biotin, Folic acid, Vitamin B12, Niacin, Pyridoxine, Ascorbic acid, Inositol and Choline. Minerals and trace elements include but are not limited to magnesium, sodium, manganese, iron, zinc, copper, selenium, phosphorous, cobalt and iodine. The addition of these components further contributes to a complete and healthy diet of the animals and positively influences their zootechnical performance. Inclusion of these components in the composition simplifies the steps needed for feeding of the animals as these components no longer need to be added separately to the feed, resulting in an improved efficiency of the feeding process. In addition, the provision of pelargonic acid or derivatives in the animals' feed was found to result in an increase in feed palatability, which in turn leads to animals that eat more, grow bigger, faster and / or healthier, which eventually leads to an increase in the profitability of the swine production.

In one embodiment, pelargonic acid (C9) is provided in liquid or solid form. The term "solid form" means a powder, a granule or a pellet in particular. The term "liquid form", in particular, means a solution in water or means a solution in oil and includes a viscous paste and a non-viscous solution. Pelargonic acid (C9) or derivatives as described above are oil-soluble and can be provided both as powder and as an oil-solution. The sodium salt of pelargonic acid (sodium nonanoate) increases the water-solubility. Use of in particular, the composition is suitable for oral administration.

A feed comprising pelargonic acid according to an embodiment of the invention is produced or manufactured by means known to a person skilled in the art. In an embodiment, the feed comprising the composition according to the invention is provided as a dry extruded feed pellet. This formulation allows a relatively long shelf life and also permits the packaging and storage of large amounts of feed.

In a further embodiment, the feed may further comprise other well-known ingredients so as to provide a nutritionally balanced complete food, including, but not limited to, plant matter, e.g., flour, meal, starch or cracked or processed grain produced from a crop plant such as wheat or other cereals, alfalfa, corn, oats, potato, rice, soybeans or other legumes; cellulose in a form that may be obtained from wood pulp, grasses, plant leaves, and waste plant matter such as rice or soy bean hulls, or corn cobs; animal matter, e.g., fish or crustacean meal, oil, protein or solubles and extracts, krill, meat meal, bone meal, feather meal, blood meal, or cracklings; algal matter; yeast; bacteria; vitamins, minerals, and amino acids; organic binders or adhesives; and chelating agents and preservatives.

Preferably, the animals are treated or fed at least once per day, more preferably two or more times per day such as, for example, 2-6 or 4-6 times per day. It is preferred that any excess food for treatment by oral administration be removed after the feeding period, e.g., by flushing out of a raceway system, or through removal out of the feed trunks.

A drinking water comprising pelargonic acid according to an embodiment is produced or manufactured by means known to a person skilled in the art and can further comprise one or more stabilizers and / or emulsifiers which improve the mixability of pelargonic acid in animal drinking water. Such stabilizers may comprise glycerol. Preferably, the composition comprises said stabilizers in a concentration of between 0,5% and 2,5% on the total weight of the composition. By preference, the composition comprises said emulsifiers in a concentration of between 10% and 20% on the total weight of the composition.

By preference, the animal treated or which is given the animal feed or drinking water is chosen from the group of cattle, poultry or pigs, preferably pigs such as is a domestic pig, or a warthog.

In a second aspect, the current invention also discloses a method of inhibiting or mitigating viral infections in an animal feed, feed ingredients or drinking water said method comprising dosing pelargonic acid (C9) to said animal feed, feed ingredient or drinking water.

Said method uses an effective amount of a chemical mitigant to inhibit viral infections from ASFV, in animal feed or drinking water, for example, to concentrations below the levels of detection through cytopathic effect (CPE), RT-PCR and/or virus isolation in cell culture. As used herein, an "effective amount" refers to an amount capable of providing bioavailable levels of the active compound (in the current case pelargonic acid )

sufficient to achieve the desired performance improvement.

Above-mentioned preferred embodiments are considered to be applicable for the purpose of inhibiting the viruses in animal feed or drinking water.

By preference, pelargonic acid is dosed in said animal feed or drinking water at a dosage of between 1 and 10000 ppm on the total weight of said animal feed or drinking water. In an embodiment, said pelargonic acid can be dosed between 50 ppm to 10.000 ppm, more preferably between 100 ppm and 10000 ppm by weight. More by preference, pelargonic acid is dosed between 200 ppm and 5000 ppm by weight, more preferably between 250 and 5000 ppm by weight, even more preferably between 250 and 3000 ppm or between 1000 and 5000 ppm by weight, such as 1250 ppm, 1500 ppm, 2000 ppm or 3750 ppm based on the total weight of said animal feed or drinking water.

As discussed above, in one or more embodiments, pelargonic acid may be mixed with one or more organic acids. For example, in one or more embodiments, a blend of two or more organic acids may be introduced to the feed or drinking water.

According to a further or another embodiment and as previously disclosed, pelargonic acid is combined with one or more organic acids chosen from the group of short chain fatty acids and their derivatives, medium chain fatty acids and their derivatives, butyric acid, propionic acid, acetic acid, lactic acid, formic acid, citric acid, oxalic acid, malic acid, or combinations thereof.

In one embodiment, pelargonic acid is combined with medium chain fatty acids (MCFAs) or their derivatives, wherein said MCFAs are chosen from the group of valeric acid (C5), caproic acid (C6), caprylic acid (C8), capric acid (C10), lauric acid (C12), derivatives thereof or combinations thereof. MCFAs for use in the present invention include valeric acid, caproic acid, caprylic acid, capric acid, pelargonic acid and/or lauric acid. Therefore, in certain embodiments, the chemical inhibitor is selected from the group consisting of valeric acid, caproic acid, caprylic acid, capric acid, lauric acid, and mixtures thereof in addition to pelargonic acid.

In a further embodiment, a blend of pelargonic acid and the sum of the other MCFAs is introduced to the feed or drinking water, at a weight ratio of between 50: 1 to 1:1, preferably 45:1 to 1:1, preferably 40:1 to 1:1, preferably 35:1 to 1:1, preferably 30:1 to 1:1, preferably 25:1 to 1:1, preferably 20:1 to 1:1, preferably 15:1 to 1:1, more preferably 10: 1 to 1:1.

It was found that an excess in pelargonic acid reinforced the action of the other MCFAs.

In one embodiment, pelargonic acid (C9) is provided in liquid or solid form. As described before, the term "solid form" means a powder, a granule or a pellet in particular. The term "liquid form", in particular, means a solution in water or means a solution in oil and includes a viscous paste and a non-viscous solution.

Consequently, in a third and fourth aspect, the current invention also discloses a method of lowering the viral titer in an animal feed, feed ingredient or drinking water, said method comprises adding an effective dose of pelargonic acid to said animal feed, feed ingredient or drinking water as well as the use of pelargonic acid (C9) for inhibiting, inactivating or eliminating viral contamination in an animal feed, feed ingredient or drinking water. It will be understood that the method as herein described preferably refers to pelargonic acid and all of its embodiments described above, and that the effects and advantages thereof equally apply to the present method.

In an embodiment, pelargonic acid or derivatives are added at a concentration of between 1 and 10000 ppm on the total weight of said animal feed or drinking water. In an embodiment, said pelargonic acid can be dosed between 50 ppm to 10.000 ppm, more preferably between 100 ppm and 10000 ppm by weight. More by preference, the composition is dosed between 200 ppm and 5000 ppm by weight, more preferably between 250 and 5000 ppm by weight, even more preferably between 250 and 3000 ppm or between 1000 and 5000 ppm by weight, such as 1250 ppm, 1500 ppm, 2000 ppm or 3750 ppm based on the total weight of said animal feed or drinking water.

The targeted viral infections are by African Swine Fever virus (ASFV).

In one embodiment, the method is used for inhibiting, inactivating or eliminating viral contamination in an animal feed, feed ingredient or drinking water. Embodiments of the present invention are particularly suitable for use in the transport of feed and feed ingredients. Feed or ingredients may become contaminated with viral infections at the point of processing, and it has been discovered that these contaminants may survive and grow for days or weeks with the feed or ingredients under conditions generally experienced during overseas transport and storage. Advantageously, embodiments of the present invention are particularly suitable to inhibit or prevent the growth of these contaminants. Therefore, in certain embodiments, methods in accordance with the present invention comprise introducing pelargonic acid to the feed or feed ingredients after processing. Pelargonic acid may be mixed with the feed or feed ingredients for sufficient time so as to provide a homogeneous mixture. In certain embodiments, methods in accordance with the present invention may prevent or decrease viral titers in feed and/or ingredients for at least about 100 days of transport and storage after processing, at least about 75 days of transport and storage after processing, at least about 50 days of transport and storage after processing, or at least about 25 days of transport and storage after processing.

In an embodiment, addition of pelargonic acid to a feed, feed ingredient or drinking water will lower the viral titer with at least 30%, at least 40%, at least 50%, at least 60%, at least 70%, at least 80%, preferably at least 85%, more preferably at least 90%, most preferably at least 95% compared to untreated feed, ingredients or drinking water.

As disclosed above, in one embodiment, pelargonic acid is added at a concentration of between 1 and 10.000 ppm to said feed, feed ingredients or drinking water. In an embodiment, said pelargonic acid can be dosed between 50 ppm to 10.000 ppm, more preferably between 100 ppm and 10000 ppm by weight. More by preference, pelargonic acid is dosed between 200 ppm and 5000 ppm by weight, between 250 and 5000 ppm by weight, more preferably between 250 and 3000 ppm by weight or between 1000 and 5000 ppm by weight, such as 1250 ppm, 1500 ppm, 2000 ppm or 3750 ppm based on the total weight of said animal feed or drinking water.

The targeted viral contamination concerns contaminations from African Swine Fever Virus (ASFV).

In one embodiment, the feed, feed ingredient or water is provided to farm animals, preferably pigs. This composition of the feed, feed ingredient or water containing pelargonic acid is effective in inactivating viruses as well as improving growth of pigs when administered orally.

In a further embodiment, the feed further comprises other well-known ingredients selected from but not limited to, the group consisting of swine feed, blood meal, porcine meat and bone meal (MBM), spray-dried animal plasma, feather meal, avian blood meal, poultry by-product meal, vitamins such as vitamin D, lysine hydrochloride, choline chloride, soybean meal, and dry net food, so as to provide a nutritionally balanced complete food. Additionally, feed can be supplemented with yeast, bacteria, minerals, and amino acids; organic binders or adhesives; and chelating agents and preservatives.

Embodiments of the present invention advantageously provide a safe alternative method of preventing or decreasing viral infections from African Swine Fever Virus (ASFV)

in animal feed or drinking water. Prior methods using harmful chemicals have displayed negative effects on protein and amino acid metabolism of animals. Unlike prior methods, the present invention uses generally non-hazardous chemical mitigants at doses discovered to achieve effective mitigation of viral infections. The chemical mitigants used in accordance with the present invention are natural alternatives that pose essentially no risk to the safety of workers or the environment. Additional advantages of the various embodiments of the invention will be apparent to those skilled in the art upon review of the disclosure herein and the working examples below.

The present invention will be now described in more details, referring to examples that are not limitative.

### EXAMPLES

The present invention will now be further exemplified with reference to the following examples.

### Example 1 (not part of the invention).

Vero cells were infected with virus particles of Porcine Reproductive and Respiratory Syndrome virus (PRRS). This virus was used as a model enveloped virus. The effect on the cells was measured by determining the cytopathic effect (CPE). The CPE was checked using an inverted microscope after 5 days post infection. Different MCFA mixtures were tested at a 0.1% concentration added to the tissue culture. Tests were duplicated. In short, virus suspension was mixed with the MCFA mixtures to be tested and immediately added to a confluent cell monolayer in a 96-well plate (a total volume of 200 µl per well was added). CPE was checked after 5 days of incubation, the results are given in Table 1. The test results for a 100% C12 solution were hampered by the fact that a solubility issue was observed, influencing the results.

Settings comprising C9 scored remarkably well in the test. While the scoring in Table 1 does not allow to distinguish between the various C9 settings, visual scoring allowed to conclude that mixtures with higher C9 concentrations were generally more protective against the cytopathic effect of the virus. Also the mixtures comprising both C8 and C9 scored remarkably well.

**Table 1**

| **MCFA mixture added** | **CPE* (first and second test)** |
|---|---|
| 100% C6 | 3/3 |
| 100 % C8 | 2/2 |
| 100 % C9 | 2/2 |
| 100 % C10 | 2/4 |
| 100 % C12 | N.A. |
| 1:1:1 C6/C8/C10 | 3/2 |
| 1:1:1:1 C6/C8/C9/C10 | 2/2 |
| 1:1 C8:C9 | 2/2 |
| Positive control (no virus particles/no MCFA) | 1/1 |
| Negative control (virus particles/no MCFA) | 4/4 |

| | |
|---|---|
| * 4= total destruction of the cells/ 3= medium destruction / 2= low destruction / 1= no destruction | |

### Example 2 (not part of the invention).

The above example was repeated but this time with PED virus inoculation. Comparable results were obtained as in example 1. C9 gave the best results in terms of protecting against viral infection and cytolytic effect of the cells.

### Example 3. The susceptibility to pelargonic acid in feed and water spiked with African Swine Fever Virus

### A. INFORMATION CONCERNING MCFA COMPOSITIONS

*1. Compositions for animal feed application*
   a. Composition F1:
      100% pelargonic acid (C9);
   b. Composition F2:
      50% pelargonic acid (C9), 25% caproic acid (C6) and 25% lauric acid (C12);
   c. Composition F3:
      95% pelargonic acid (C9) and 5% caprylic acid (C8).
*2. Compositions for drinking water application*
   a. Composition W1:
      14% pelargonic acid, 15% emulsifiers, 70% organic acids and 1% glycerol
   b. Composition W2:
      14% of a mixture consisting of 50% pelargonic acid (C9), 25% caproic acid (C6) and 25% lauric acid (C12), 15% emulsifiers, 70% organic acids and 1% glycerol, MCFAs consisting of 50% pelargonic acid (C9), 25% caproic acid (C6) and 25% lauric acid (C12);
   c. Composition W3:
      14% of a mixture consisting of 95% pelargonic acid (C9) and 5% caprylic acid (C8), 15% emulsifiers, 70% organic acids and 1% glycerol, MCFAs.

### B. INFORMATION CONCERNING AFRICAN SWINE FEVER VIRUS (ASFV)

The ASFV strain used for all experiments was isolated in the Red River Delta Region (sample from Hanoi, Vietnam). This strain has been confirmed by virus isolation, HAD assay and real-time PCR as recommended by OIE (the World Organization for Animal Health).

The ASFV strain as used herein has the following genetic characterization, belonging to genotype II, serotype VIII and contain a TRS gene, indicating that it is very close and thus considered representative to ASFV strains detected in China and Belgium, 2018 and differs from ASFV isolated in Georgia in 2007. The Virus Stock Titration used herein equals 10⁷ HAD₅₀/mL (heme adsorbing dose).

### C. EXPERIMENTAL DESIGN

### 1. Animal feed experimental design

### Control groups

Treatment of the feed with animal feed compositions before contamination with ASFV.
- Positive control: complete feed containing 10⁵ HAD₅₀/g of ASFV
- Negative control: complete feed, no ASFV added
- Sampling time-point: after 24-hour exposure
- Incubation of the samples at room temperature (lab condition) for 24 hours
- Evaluated by real-time PCR based on OIE protocol

### Experimental groups

- Feed experiment 1
   Composition F1 treated feed at different doses (1250, 2500, 3750 and 5000 ppm) will be spiked with ASFV and final virus titration is 10⁵ HAD₅₀/g of feed.
- Feed experiment 2
   Composition F2 treated feed at different doses (1250, 2500, 3750 and 5000 ppm) will be spiked with ASFV and final virus titration is 10⁵ HAD₅₀/g of feed.
- Feed experiment 3
   Composition F3 treated feed at different doses (1250, 2500, 3750 and 5000 ppm) will be spiked with ASFV and final virus titration is 10⁵ HAD₅₀/g of feed.

### 2. Drinking water experimental design

### Control groups

Treatment of the drinking water with drinking water compositions before contamination with ASFV.
- Positive control: fresh water treated with ASFV isolated in Vietnam at different doses, 10¹ HAD₅₀, 10² HAD₅₀ and 10³ HAD₅₀
- Negative control: fresh water (ASFV negative by real-time PCR)
- Sampling time-point: 30 min, 1, 3, 6, 12 and 24 hours after virus incubation at room temperature
- Evaluated by real-time PCR based on OIE protocol

### Experimental groups

- Water experiment 1
   Composition W1 treated water at different doses (1250, 2500, 3750 and 5000 ppm) will be spiked with ASFV and final virus titration is 10¹ HAD₅₀, 10² HAD₅₀ and 10³ HAD₅₀/mL
- Water experiment 2
   Composition W2 treated water at different doses (1250, 2500, 3750 and 5000 ppm) will be spiked with ASFV and final virus titration is 10¹ HAD₅₀, 10² HAD₅₀ and 10³ HAD₅₀/mL
- Water experiment 3
   Composition W3 treated water at different doses (1250, 2500, 3750 and 5000 ppm) will be spiked with ASFV and final virus titration is 10¹ HAD₅₀, 10² HAD₅₀ and 10³ HAD₅₀/mL

### 3. Product treatment

**Animal feed treatment** (Kansas State University, 2019)
- Virus working stock of 10⁶ HAD₅₀/ml was used
- To ensure a homogeneous spread of the compositions on the feed, 100g of complete feed was treated with each composition at different doses (1250, 2500, 3750 and 5000 ppm
- Consequently, 22.5g of treated feed was placed in a glass bottle (250 mL) and kept at room temperature for 24h
- 2.5 mL of virus working stock was added to each bottle, obtaining a final virus titration of 10⁵HAD₅₀/g of feed
- The samples were incubated at room temperature for 24 hours
- Sample preparation: 100 mL PBS buffer was placed in each bottle containing 22.5 g of treated feed, 200 µL of supernatant from each sample was used for DNA extraction using QIAamp DNA Mini Kit
- Real-time PCR was performed based on the OIE protocol

### Drinking water treatment

- Virus working stock of 10⁴, 10³ and 10² HAD₅₀/ml was used
- 200 mL of fresh water was treated with each composition at different doses (1250, 2500, 3750 and 5000 ppm)
- Treated water was vortexed and kept at room temperature for 24h
- 22.5 mL of treated water was placed in 50 ml tubes, each tube was spiked with 2.5 mL of 10⁴, 10³ and 10² HAD₅₀ of ASFV, obtaining a final virus titration of 10³ HAD₅₀, 10² HAD₅₀ and 10¹ HAD₅₀/mL of water
- Sample preparation: at time-points 30 min, 1, 3, 6, 12 and 24 hours after virus incubation, samples were collected for DNA extraction and DNA was stored at -80°C until real-time PCR
- Real-time PCR was performed based on the OIE protocol

### 4. DNA preparation

For DNA preparation, a QIAamp DNA Mini Kit was used (Qiagen).

### 5. Real-time PCR

Real-time PCR was employed to evaluate the effect of MCFA on ASFV isolated in Vietnam, based on the OIE protocol "SOP/CISA/ASF/PR/2 Standard operation procedure for the detection of African Swine Fever Virus (ASFV) by real-time polymerase chain reaction (PCR)", 2018. Results of the real-time PCR protocol are expressed in quantification cycles (Cq) required to detect viral nucleic acid. Hence, high Cq values need to be interpreted in the sense that less viral nucleic acid is present.

### 6. Statistical analysis

Statistical analysis was performed using IBM SPSS software (SPSS 23.0 for Windows; IBM, Chicago, IL, USA). A p-value < 0.05 was considered to be statistically significant. Differences among the groups were tested by Duncan's multiple comparison methods.

### D. RESULTS

### 1. Susceptibility of African Swine Fever Virus (ASFV) to medium chain fatty acids (MCFAs) in animal feed

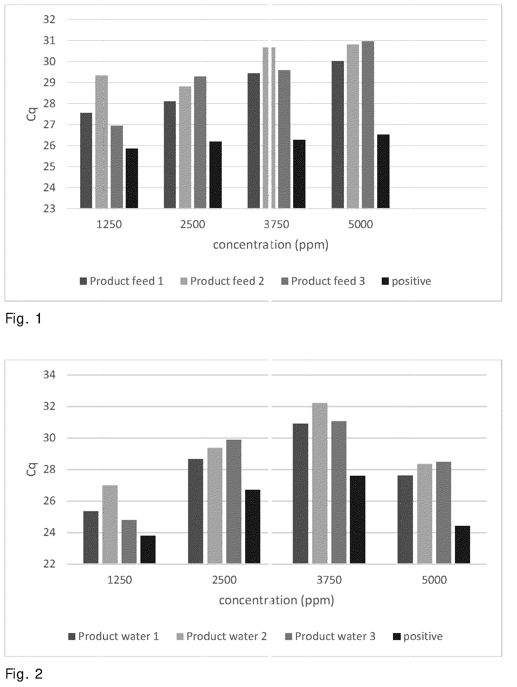

Results of the experiments described above concerning treated animal feed are shown in Figure 1. Results demonstrate that all compositions, including F1, F2 and F3, significantly increase the Cq value for ASFV when compared to the positive control (P<0.01) at highest doses of 3750 and 5000 ppm.

Composition F1 shows particularly effective as all doses (1250, 2500, 3750 and 5000 ppm) induced a statistical increase in Cq value when compared to positive control (P<0.05). In lower doses, composition F2 with a concentration of 2500 ppm significantly enhanced the Cq value when compared to positive control (P<0.05). From the results as herein discussed, it can be concluded that all tested compositions for feed treatment show significant efficacy for inhibiting/reducing ASFV in animal feed.

### 2. Susceptibility of African Swine Fever Virus (ASFV) to medium chain fatty acids (MCFAs) in drinking water

Results of the experiments described above concerning treated drinking water are shown in Figure 2. Results demonstrate that all compositions, including W1, W2 and W3, significantly increase the Cq value for ASFV when compared to the positive control (P<0.01) at highest doses of 3750 and 5000 ppm.

Composition W1 shows particularly effective as all doses (1250, 2500, 3750 and 5000 ppm) induced a statistical increase in Cq value when compared to positive control (P<0.05). In lower doses, composition W2 with a concentration of 2500 ppm significantly enhanced the Cq value when compared to positive control (P<0.05). The present invention is in no way limited to the embodiments described in the examples and/or shown in the figures. On the contrary, methods according to the present invention may be realized in many different ways without departing from the scope of the invention.

### Example 4.

. Inhibition of ASF viral load by C9 in acid vs. ester (monoglyceride) form Animal feed was infected with ASF, at a titer of 10⁵ and treated with 0.1 % C9 in either acid form (nonanoic acid) or monoglyceride form (nonanoic ester). In the control group, no treatment was applied after the viral inoculation. The samples were incubated for 12h at room temperature and evaluated by real-time PCR for monitoring the level of ASF. The PCR cycle number (Cq) at which the reaction curve of each sample intersects the threshold line was determined. The results are depicted in Table 2. The treatment of the feed with C9 in acid form significantly reduced the viral load of ASF (Cq 28.4) when compared with the treatment with C9 in ester form (Cq 26.3) or untreated feed (Cq 25.9).

**Table 2**

| **Treatment** | **Cq value of ASF after 12h of incubation** |
|---|---|
| Control | 25.9 |
| Nonanoic acid | 28.4* |
| Nonanoic ester (monoglyceride) | 26.3 |

| | |
|---|---|
| *P<0.05 | |

## Claims

1. Pelargonic acid (C9) for use in the treatment or prevention of African Swine Fever virus (ASFV) infections in animals from the family Suidae.

2. Pelargonic acid (C9) for use according to claim 1, wherein said pelargonic acid is added to the feed, feed ingredients or the drinking water of livestock.

3. Pelargonic acid (C9) for use according to any of the previous claims, wherein said pelargonic acid is added at a concentration of between 1 and 10.000 ppm to said feed, feed ingredients or drinking water.

4. Pelargonic acid (C9) for use according to any of the previous claims, wherein said pelargonic acid is combined with one or more other organic acids.

5. Pelargonic acid (C9) for use according to claim 4, wherein said organic acids are chosen from short chain fatty acids, medium chain fatty acids, propionic acid, butyric acid, acetic acid, lactic acid, formic acid, citric acid, oxalic acid, malic acid, or combinations thereof.

6. Pelargonic acid (C9) for use according to claim 4 wherein said pelargonic acid is combined with medium chain fatty acids (MCFAs), wherein said MCFAs are chosen from the group of valeric acid (C5), caproic acid (C6), caprylic acid (C8), capric acid (C10), lauric acid (C12), or combinations thereof.

7. Pelargonic acid (C9) for use according to claim 6, wherein the ratio between C9 and the sum of the other MCFAs is between 50:1 to 1:1, more preferably 10:1 to 1:1.

8. Pelargonic acid (C9) for use according to any of the previous claims wherein said pelargonic acid (C9) is provided in liquid or solid form.

9. Pelargonic acid (C9) for use according to any of the previous claims, wherein said animal from the family Suidae is a domestic pig or a warthog.

10. A method of inhibiting or mitigating ASFV infections in an animal feed, feed ingredients or drinking water said method comprising dosing pelargonic acid (C9) to said animal feed, feed ingredient or drinking water.

11. The method according to claim 10, wherein said pelargonic acid are added at a concentration of between 1 and 10.000 ppm to said feed, feed ingredients or drinking water.

12. The method according to claim 10 or 11, wherein said pelargonic acid is combined with one or more other organic acids.

13. The method according to claim 12, wherein said organic acids are chosen from short chain fatty acids, medium chain fatty acids **,** propionic acid, acetic acid, lactic acid, formic acid, citric acid, oxalic acid, malic acid, or combinations thereof.

14. A method of lowering the viral titer of ASV in an animal feed, feed ingredient or drinking water, said method comprises adding an effective dose of pelargonic acid to said animal feed, feed ingredient or drinking water.

15. The method according to claim 14, wherein said pelargonic acid is added at a concentration of between 1 and 10.000 ppm to said feed, feed ingredients or drinking water.

## Patentansprüche

1. Pelargonsäure (C9) zur Verwendung bei der Behandlung oder Prävention von Infektionen mit dem Virus der Afrikanischen Schweinepest (ASF) bei Tieren der Familie echter Schweine.

2. Pelargonsäure (C9) zur Verwendung nach Anspruch 1, wobei die Pelargonsäure dem Futter, den Futterbestandteilen oder dem Trinkwasser des Viehbestandes zugesetzt wird.

3. Pelargonsäure (C9) zur Verwendung nach einem der vorhergehenden Ansprüche, wobei die Pelargonsäure dem Futter, den Futterbestandteilen oder dem Trinkwasser in einer Konzentration zwischen 1 und 10.000 ppm zugesetzt wird.

4. Pelargonsäure (C9) zur Verwendung nach einem der vorhergehenden Ansprüche, wobei die Pelargonsäure mit einer oder mehreren anderen organischen Säuren kombiniert wird.

5. Pelargonsäure (C9) zur Verwendung nach Anspruch 4, wobei die organischen Säuren aus kurzkettigen Fettsäuren, mittelkettigen Fettsäuren, Propionsäure, Buttersäure, Essigsäure, Milchsäure, Ameisensäure, Zitronensäure, Oxalsäure, Äpfelsäure oder Kombinationen daraus ausgewählt werden.

6. Pelargonsäure (C9) zur Verwendung nach Anspruch 4, wobei die Pelargonsäure mit mittelkettigen Fettsäuren (MCFAs) kombiniert wird, wobei die MCFAs aus der Gruppe der Valeriansäure (C5), der Capronsäure (C6), der Caprylsäure (C8), der Caprinsäure (C10), der Laurinsäure (C12) oder Kombinationen daraus ausgewählt werden.

7. Pelargonsäure (C9) zur Verwendung nach Anspruch 6, wobei das Verhältnis zwischen C9 und der Summe der anderen MCFAs zwischen 50:1 und 1:1 liegt, bevorzugter zwischen 10:1 und 1:1.

8. Pelargonsäure (C9) zur Verwendung nach einem der vorhergehenden Ansprüche, wobei die Pelargonsäure (C9) in flüssiger oder fester Form bereitgestellt wird.

9. Pelargonsäure (C9) zur Verwendung nach einem der vorhergehenden Ansprüche, wobei das Tier der Familie der echten Schweine ein Hausschwein oder ein Warzenschwein ist.

10. Verfahren zum Hemmen oder Abschwächen von Infektionen mit dem ASF-Virus in einem Tierfutter, einem Futterbestandteil oder dem Trinkwasser, wobei das Verfahren das Dosieren von Pelargonsäure (C9) in das Futter, den Futterbestandteil oder das Trinkwasser umfasst.

11. Verfahren nach Anspruch 10, wobei die Pelargonsäure dem Futter, den Futterbestandteilen oder dem Trinkwasser in einer Konzentration zwischen 1 und 10.000 ppm zugesetzt wird.

12. Verfahren nach Anspruch 10 oder 11, wobei die Pelargonsäure mit einer oder mehreren anderen organischen Säuren kombiniert wird.

13. Verfahren nach Anspruch 12, wobei die organischen Säuren aus kurzkettigen Fettsäuren, mittelkettigen Fettsäuren, Propionsäure, Essigsäure, Milchsäure, Ameisensäure, Zitronensäure, Oxalsäure, Maleinsäure oder Kombinationen daraus ausgewählt werden.

14. Verfahren zum Senken des Virustiters von ASF in einem Tierfutter, Futterbestandteil oder dem Trinkwasser, wobei das Verfahren das Zusetzen einer wirksamen Dosis Pelargonsäure zu dem Tierfutter, Futterbestandteil oder dem Trinkwasser umfasst.

15. Verfahren nach Anspruch 14, wobei die Pelargonsäure dem Futter, den Futterbestandteilen oder dem Trinkwasser in einer Konzentration zwischen 1 und 10.000 ppm zugesetzt wird.

## Revendications

1. Acide pélargonique (C9) destiné à être utilisé pour le traitement ou la prévention des infections par le virus de la peste porcine africaine (VPA) chez les animaux de la famille des suidés.

2. Acide pélargonique (C9) destiné à être utilisé selon la revendication 1, dans lequel ledit acide pélargonique est ajouté aux aliments, aux ingrédients d'aliments ou à l'eau de boisson des animaux.

3. Acide pélargonique (C9) destiné à être utilisé selon l'une quelconque des revendications précédentes, dans lequel ledit acide pélargonique est ajouté à une concentration comprise entre 1 et 10 000 ppm auxdits aliments, ingrédients d'aliments ou eau de boisson.

4. Acide pélargonique (C9) destiné à être utilisé selon l'une quelconque des revendications précédentes, dans lequel ledit acide pélargonique est combiné à un ou plusieurs autres acides organiques.

5. Acide pélargonique (C9) destiné à être utilisé selon la revendication 4, dans lequel lesdits acides organiques sont choisis parmi les acides gras à chaîne courte, les acides gras à chaîne moyenne, l'acide propionique, l'acide butyrique, l'acide acétique, l'acide lactique, l'acide formique, l'acide citrique, l'acide oxalique, l'acide malique, ou des combinaisons de ceux-ci.

6. Acide pélargonique (C9) destiné à être utilisé selon la revendication 4, dans lequel ledit acide pélargonique est combiné à des acides gras à chaîne moyenne (AGCM), dans lequel lesdits AGCM sont choisis dans le groupe constitué par acide valérique (C5), acide caproïque (C6), acide caprylique (C8), acide caprique (C10), acide laurique (C12), ou des combinaisons de ceux-ci.

7. Acide pélargonique (C9) destiné à être utilisé selon la revendication 6, dans lequel le rapport entre le C9 et la somme des autres AGCM est compris entre 50:1 et 1:1, de préférence entre 10:1 et 1:1.

8. Acide pélargonique (C9) destiné à être utilisé selon l'une quelconque des revendications précédentes, dans lequel ledit acide pélargonique (C9) est fourni sous forme liquide ou solide.

9. Acide pélargonique (C9) destiné à être utilisé selon l'une quelconque des revendications précédentes, dans lequel ledit animal de la famille des suidés est un porc domestique ou un phacochère.

10. Procédé d'inhibition ou d'atténuation des infections par VPA dans des aliments pour animaux, des ingrédients d'aliments ou de l'eau de boisson, ledit procédé comprenant le dosage d'acide pélargonique (C9) dans les aliments pour animaux, les ingrédients d'aliments ou l'eau de boisson.

11. Procédé selon la revendication 10, dans lequel ledit acide pélargonique est ajouté à une concentration comprise entre 1 et 10 000 ppm auxdits aliments, ingrédients d'aliments ou eau de boisson.

12. Procédé selon la revendication 10 ou 11, dans lequel ledit acide pélargonique est combiné à un ou plusieurs autres acides organiques.

13. Procédé selon la revendication 12, dans lequel lesdits acides organiques sont choisis parmi les acides gras à chaîne courte, les acides gras à chaîne moyenne, l'acide propionique, l'acide acétique, l'acide lactique, l'acide formique, l'acide citrique, l'acide oxalique, l'acide malique, ou des combinaisons de ceux-ci.

14. Procédé de réduction du titre viral de I'VPA dans un aliment pour animaux, un ingrédient d'aliment ou de l'eau de boisson, ledit procédé comprend l'ajout d'une dose efficace d'acide pélargonique à l'aliment pour animaux, à l'ingrédient d'aliment ou à l'eau de boisson.

15. Procédé selon la revendication 14, dans lequel ledit acide pélargonique est ajouté à une concentration comprise entre 1 et 10 000 ppm auxdits aliments, ingrédients d'aliments ou eau de boisson.
